# EUROPÄISCHE PATENTSCHRIFT

(11) **EP 3 117 847 B1**
(45) Veröffentlichungstag und Bekanntmachung des Hinweises auf die Patenterteilung: **10.03.2021**
(21) Anmeldenummer: 16176879.1
(22) Anmeldetag: 29.06.2016
(51) Int. Cl.: A61M 1/16

(54) **EXTRAKORPORALE BLUTBEHANDLUNGSANLAGE MIT INTEGRIERTEM DESINFEKTIONSBEHÄLTER**
EXTRACORPOREAL BLOOD PROCESSING APPARATUS WITH INTEGRATED DISINFECTION TANK
INSTALLATION DE TRAITEMENT DE SANG EXTRACORPOREL COMPRENANT UN RECIPIENT DE DESINFECTION INTEGRE

(30) Priorität: 14.07.2015 DE 202015103684 U
(43) Veröffentlichungstag der Anmeldung: 18.01.2017
(73) Patentinhaber: B. Braun Avitum AG, 34212 Melsungen (DE)
(72) Erfinder: Thore, Heinemann, 34320 Söhrewald-Wellerode (DE)
(74) Vertreter: Winter, Brandl, Fürniss, Hübner, Röss, Kaiser, Polte - Partnerschaft mbB

(56) Entgegenhaltungen:
- DE-A1- 2 559 241
- DE-A1- 4 138 140
- DE-A1-102007 022 547
- DE-U1- 7 812 762
- DE-U1- 29 503 691
- US-A- 4 366 051
- US-A1- 2005 000 828

## Beschreibung

Die vorliegende Erfindung betrifft eine extrakorporale Blutbehandlungsanlage, insbesondere eine Dialyseanlage, gemäß dem Oberbegriff des Anspruchs 1, mit mindestens einer extrakorporalen Blutbehandlungsmaschine, wie einer Dialysemaschine, welche unter anderem mittels Anschlüssen, Leitungen und einer entsprechenden Steuerung dazu vorbereitet ist, nach einem Blutreinigungsprozess einen zentral gesteuerten Desinfektionsprozess zu durchlaufen.

### Hintergrund der Erfindung

In extrakorporalen Blutbehandlungsanlagen, vorzugsweise Blutreinigungsanlagen, wie Dialyseanlagen, sind mindestens eine, meist mehrere Blutbehandlungs- / Blutreinigungs- / Dialysemaschinen angeordnet. Die einzelnen Dialysemaschinen und die zugehörigen Komponenten durchlaufen nach jedem Dialyse-Arbeitszyklus, der aus verschiedenen Betriebsphasen zusammengesetzt ist, einen Desinfektionsprozess. Bei diesem Desinfektionsprozess werden sämtliche Teile der Dialyseanlage, die während des Arbeitszyklus unmittelbar oder auch mittelbar in Kontakt mit Fluiden, wie Blut oder Dialyseflüssigkeit, standen, die demnach kontaminiert sind, desinfiziert. Zu jenen Teilen zählen beispielsweise sämtliche Fluidleitungen, Wärmetauscher und Ventile, die während des Arbeitszyklus Fluide transportierten bzw. beförderten bzw. mit diesen in Kontakt standen.

Weiterhin werden nach dem Dialyse-Arbeitszyklus auch Gegenstände desinfiziert, die während des Dialyseprozesses als Hilfsgegenstände dienten. Zu diesen Hilfsgegenständen zählen Instrumente/Teile/Mittel/Materialien, die über die Dialysemaschine hinaus für eine Durchführung des Dialyseprozesses notwendig sind. Als Beispiele für sie seien an dieser Stelle Scheren, Klemmen, wie Schlauchklemmen, einzelne Bauelemente des Dialysefilters, Klappmechanismen, Schlauch-/Leitungsanschlüsse und Abdeckfolien erwähnt. Das Spektrum der Hilfsgegenstände ist jedoch keineswegs auf die genannten Teile zu reduzieren, vielmehr stellt diese Auswahl einige wenige Beispiele vor.

Das bekannte Konzept zur Desinfektion dieser Hilfsgegenstände beruht auf einem vom Desinfektionsprozess der Dialyseanlage entkoppelten Verfahren. Hierfür ist es nötig, sämtliche zu desinfizierenden Hilfsgegenstände nach Ablauf des Arbeitszyklus manuell an ein von der Dialyseanlage separates Desinfektionsgerät zu übergeben. Diese separate Desinfektion der Hilfsgegenstände, die unabhängig von der Desinfektion der Dialyseanlage von Statten geht, zieht einen zusätzlichen Prozessschritt und somit ein gesteigertes Maß an Komplexität und Kosten nach sich.

### Stand der Technik

Die im vorstehenden Abschnitt erwähnte Desinfektion der Hilfsgegenstände in einem separaten Desinfektionsgerät kann auf verschiedene Arten und Weisen realisiert werden. Ein verbreitetes Vorgehen besteht darin, die Hilfsgegenstände händisch unter Verwendung von Desinfektionshandschuhen und -mitteln zu desinfizieren. Ein weiteres Verfahren zeichnet sich durch den Einsatz eines Desinfektionsbehälters aus. Dieser besitzt das Merkmal, dass er Hilfsgegenstände problemlos aufnimmt. Weiterhin weist er eine solche Gestaltung auf, dass er ohne großen Aufwand mit einem Desinfektionsmittel befüllbar und auch wieder entleerbar ist. So werden die zu desinfizierenden Gegenstände in einem solchen Desinfektionsbehälter eingeschlossen, bevor das separate Desinfektionsgerät dafür sorgt, dass dem Desinfektionsbehälter ein Desinfektionsmittel zugeführt wird. Das Desinfektionsmittel bewirkt bei einer bestimmten Temperatur und Menge, dass die im Desinfektionsbehälter befindlichen Hilfsgegenstände das gewünschte Maß an Desinfektion erreichen.

Gattungsgemäße Desinfektionsbehälter sind in den deutschen Gebrauchsmustern DE 295 03 691 U1, GM 78 14 376 U1 sowie GM 78 12 762 U1 offenbart. Darüber hinaus beschreibt die deutsche Offenlegungsschrift DE 10 2007 044 647 A1 eine Vorrichtung zum Steuern eines Fluidverlaufs. Sie ist auf dem Gebiet der Desinfektion von Dialyseanlagen einzuordnen, da darin ein Desinfektionselement offenbart ist, das an einen Katheterkonnektor anschließbar ist und durch das ein Lösungsaustausch von Dialysemitteln stattfindet. Sie trägt somit zur Hygiene der Dialyseeinheit bei, ohne jedoch den eingangs erwähnten zusätzlichen Prozessschritt der separaten Desinfektion der Hilfsgegenstände zu vermeiden. Gleiches gilt für die beiden Vorrichtungen, die in den Offenlegungsschriften DE 196 40 840 A1 und CN 102293494 (A) offenbart sind.

Weiterer gattungsgemäßer Stand der Technik ist aus den Dokumenten US 4,366,051 und DE 25 59 241 A1 sowie aus US2005000828 bekannt.

### Kurzbeschreibung der Erfindung

Der vorliegenden Erfindung liegt angesichts dieses Standes der Technik die Aufgabe zugrunde, eine Vorrichtung bereitzustellen, die den zusätzlichen Prozessschritt der separaten Desinfektion/Reinigung der Hilfsgegenstände wegfallen lässt, ohne Einbußen hinsichtlich der Güte der Desinfektion der Hilfsgegenstände in Kauf zu nehmen.

Die vorstehende Aufgabe wird durch eine Blutbehandlungsanlage mit den Merkmalen des Anspruchs 1 gelöst. Vorteilhafte Ausgestaltungen sind Gegenstand der Unteransprüche.

Die Blutbehandlungsanlage (wie eine Dialyseanlage) weist mehrere Komponenten auf. Dazu zählen erfindungsgemäß mindestens eine Blutbehandlungsmaschine (wie eine Dialysemaschine), mehrere Anschlüsse, über die ein Fluid, wie ein Desinfektionsmittel, austauschbar ist, und ein interner oder externer Desinfektionsbehälter/Sterilisationsbehälter zur Aufnahme von medizinischen Gerätschaften.

Die Blutbehandlungsanlage hat zudem ein beispielsweise innerhalb der Blutreinigungsmaschine angeordnetes, internes Fluidleitungssystem, das mehrere Leitungen aufweist, die zumindest während eines Desinfektionsprozesses von einem Desinfektionsmittel durchfließbar sind.

Außerdem hat die erfindungsgemäße Anlage den vorstehend bereits genannten Desinfektionsbehälter, der zumindest während des Desinfektionsprozesses über mindestens einen Desinfektionsbehälteranschluss derart an das interne Fluidleitungssystem anschließbar bzw. zumindest temporär angeschlossen ist, dass er während des Desinfektionsprozesses mitdesinfizierbar ist bzw. mitdesinfiziert wird. Der Desinfektionsbehälter eignet sich dafür bzw. ist dafür vorgesehen, solche zusätzlichen Hilfsgegenstände aufzunehmen, welche desinfiziert werden sollen.

Somit ist garantiert, dass die Hygienestandards, denen die Hilfsgegenstände unterliegen, weiterhin erfüllt werden, da das grundsätzliche Vorgehen der Desinfektion mittels eines Desinfektionsbehälters, wie eingangs erwähnt, bereits bekannt ist und sich bewährt hat. Es ist sogar von einer Erhöhung des Hygienefaktors auszugehen, da einerseits der Transport der Hilfsgegenstände - über teilweise längere Strecken - entfällt und andererseits dieselben, auf die jeweiligen Bedingungen optimal angepassten Desinfektionsmittel verwendet werden, wie für die restliche Dialyseanlage. Weiterhin unterliegt nun auch die Desinfektion der Hilfsgegenstände einer im weiteren Verlauf der Anmeldung näher beschriebenen, zentralen Steuereinheit, womit auch hinsichtlich ihrer Steuerung eine Abhängigkeit der beiden zuvor unabhängigen/separaten Desinfektionen hergestellt ist.

Eine ebenfalls im Laufe dieses Anmeldungstextes präzisierte, erfindungsgemäße konstruktive Gestaltung des Desinfektionsbehälters ermöglicht es zudem, den Desinfektionsbehälter an der Blutreinigungsmaschine anzubringen bzw. die beiden Komponenten "Desinfektionsbehälter" und "Dialysemaschine" vorzugsweise starr zu koppeln. Dieses Merkmal trägt dazu bei, den zusätzlichen Prozessschritt der separaten Desinfektion der Hilfsgegenstände entfallen zu lassen, da diese nun im Rahmen des zentral gesteuerten Desinfektionsprozesses der Dialysemaschine desinfiziert werden.

Als Folge aus diesen erfindungsgemäßen Änderungen ergibt sich unter anderem eine Zeitersparnis gegenüber den herkömmlichen Lösungen. Neben der gesteigerten zeitlichen Effizienz zieht das Merkmal des Anspruchs 1 eine Verringerung des Stromverbrauchs nach sich, wodurch der thermische Wirkungsgrad der Anlage steigt. Dies ist damit begründet, dass nun anstatt zweier Desinfektionsanlagen nur noch eine in Betrieb zu halten ist. Als Konsequenz daraus sinken die Betriebskosten des Dialysezentrums. Zusätzlich wird daran erinnert, dass auch der Hygienefaktor steigt.

Es kann demnach von einer Prozessverbesserung des gesamten Dialysezentrums gesprochen werden, was den Wert der vorliegenden Erfindung verdeutlicht.

So besteht eine weitere vorteilhafte Ausführungsform darin, dass die Blutbehandlungsanlage/Dialyseanlage eine zentrale elektronische Steuereinheit aufweist, die mit der Blutreinigungsmaschine elektrisch in Verbindung steht und somit über Informationen über deren momentane Betriebsphase verfügt, und welche die Blutreinigungsmaschine mit dem bereits daran fluidangeschlossenen Desinfektionsbehälter derart ansteuert, dass der Desinfektionsprozess optimal an die zu desinfizierenden Komponenten einschließlich des Desinfektionsbehälters und dessen Raumvolumen angepasst ist. Somit kann ein leistungsfähiger Hauptprozessor (CPU) dafür eingesetzt werden, die jeweiligen Parameter während eines Desinfektionsprozesses der einzelnen Komponenten optimal zu steuern. Zu jenen Parametern zählen unter anderem die Temperatur des Desinfektionsmittels, die dem Fluid mittels Wärmeübertragern zugeführt wird, die jeweilige Durchflussmenge des Hydraulikmittels durch regelbare Durchflussventile, die Einwirk-/Verweilzeiten, während derer das Desinfektionsmittel nicht zirkuliert sowie die Gesamtdauer des Desinfektionsprozesses. Die Einwirkzeiten kommen im weiteren Verlauf des Anmeldetextes noch einmal im Zusammenhang mit Retentionszeiten zur Sprache. Die Verwendung einer zentralen Steuereinheit ermöglicht es weiterhin, auf unvorhergesehene Ereignisse bei der Desinfektion zu reagieren. So können sämtliche Parameter, von denen eine kleine Auswahl im vorigen Absatz angeführt wurde, bei Bedarf variiert werden. Dies zieht ein enormes Maß an Flexibilität nach sich, was den Wert der Erfindung weiter erhöht.

Da erfindungsgemäß eine Behälterzufuhrleitung und eine Behälterabfuhrleitung des Desinfektionsbehälters mit einer einen Dialysator der Blutbehandlungsmaschine wahlweise brückenden Spülbrücke verbindbar sind, welche ein integraler Bestandteil der Blutreinigungsmaschine ist und welche mit dem internen Fluidleitungssystem in Verbindung steht, zieht dies positive Aspekte nach sich. Denn auf diese Weise wird ein sicherer Anschluss des Desinfektionsbehälters an die Desinfektionsmittelversorgung sichergestellt. Die Spülbrücke ist im Allgemeinen ein Bestandteil der Dialysemaschine. Somit ist für sie kein zusätzlicher konstruktiver Aufwand zu betreiben. Weiterhin sind beispielsweise mit Hansen-Kupplungen bereits Standardkomponenten vorhanden, um einen sicheren, strömungsverlustarmen und zeitgleich wirtschaftlichen Anschluss der erfindungsgemäßen Behälterzufuhrleitung und der Behälterabfuhrleitung mit der Spülbrücke zu realisieren.

Mittels der Möglichkeit der Versorgung des Desinfektionsbehälters über die Spülbrücke wird zudem eine Lösung präsentiert, die es zulässt, vorhandene Blutbehandlungsanlagen/Dialyseanlagen so nachzurüsten, dass sie der erfindungsgemäßen Vorrichtung entsprechen. Diese Ausführungsform birgt zudem den Vorteil, dass der Desinfektionsbehälter optional abnehmbar von seiner Desinfektionsmittelversorgung gestaltet sein kann.

Eine weitere vorteilhafte, eventuell alternative Ausführungsform zeichnet sich dadurch aus, dass die Behälterzufuhrleitung und die Behälterabfuhrleitung des Desinfektionsbehälters einen Kreislauf mit einer eigenen, mit dem internen Fluidleitungssystem in Verbindung stehenden Desinfektionsmittelversorgung bilden können. Mittels dieser Desinfektionsmittelversorgung ist eine autarke Versorgung des Desinfektionsbehälters sichergestellt. Jene Desinfektionsmittelversorgung kann somit optimal an die Anforderungen des Desinfektionsbehälters angepasst werden. Es ist zu beachten, dass die Steuerung des Desinfektionsmittelkreislaufes des Desinfektionsbehälters weiterhin von der zentralen Steuereinheit vorgenommen wird. Die in diesem Zusammenhang angesprochenen Vorteile hinsichtlich der Flexibilität und der Effizienz liegen selbstverständlich auch in dieser Ausführungsform vor.

Ebenfalls von Vorteil ist es, wenn der Desinfektionsbehälter solche Verbindungselemente aufweist, dass er starr mit der Blutreinigungsmaschine koppelbar ist. Dabei wird besonders darauf Wert gelegt, dass die Verbindungselemente hinsichtlich ihrer Gestaltung derart beschaffen sind, dass sie eine möglichst simple Montage des Desinfektionsbehälters an der Blutreinigungsmaschine zulassen. Hierfür sind vor allem formschlüssige und/oder reibkraftschlüssige Verbindungen vorgesehen. Neben diesen ist weiterhin der Einsatz von stoffschlüssigen Verbindungen denkbar. Der Einsatz von Verbindungselementen, die mit vorhandenen Blutreinigungsmaschinen kompatibel sind, ermöglicht auch hier eine Nachrüstung vorhandener Anlagen auf die erfindungsgemäße Vorrichtung. Konstruktiv ist dabei vorgesehen, den Desinfektionsbehälter seitlich an einer vorhandenen Dialysemaschine anzubringen. So liegt ein entscheidender Vorteil der Erfindung darin, dass vorhandene Anlagen weiterverwendet werden können, womit kein hoher Wertverlust von Altanlagen, die noch nicht vollständig abgeschrieben sind, eintritt. Weiterhin ist der zu stemmende Anteil an Investitionen für eine Umrüstung auf die erfindungsgemäße Vorrichtung aus den genannten Gründen überschaubar.

Eine weitere vorteilhafte Ausführungsform ist dadurch gekennzeichnet, dass der Desinfektionsbehälter integral mit der Blutreinigungsmaschine ausgebildet ist. Daraus resultiert, dass der Desinfektionsbehälter räumlich ideal an die an ihn gestellten Anforderungen angepasst ist, während die Blutreinigungsmaschine hinsichtlich ihres Bauraums und ihrer funktionalen Anordnung optimiert werden kann. Die Integration des Desinfektionsbehälters in die Blutreinigungsmaschine bewirkt darüber hinaus, dass die Anschlüsse für die Behälterzufuhrleitung und die Behälterabfuhrleitung hinsichtlich ihrer Position in bestmöglicher Abstimmung mit den anderen Anschlüssen der Blutreinigungsmaschine ausgestaltet sind. So ergeben sich mittels der Integration des Desinfektionsbehälters in die Blutreinigungsmaschine vor allem strukturelle Vorteile, die die Handhabung, wie die Ansteuerung und das Anschließen, der Blutreinigungsanlage vereinfachen.

Wenn der Desinfektionsbehälter einen Deckelsensor hat, der dazu vorbereitet ist, zu erfassen, ob ein Behälterdeckel fluiddicht auf einem Behälterkörper anliegt, sich der Behälterdeckel also in einem geschlossenen Zustand befindet oder ob der Behälterdeckel nicht fluiddicht auf dem Behälterkörper anliegt, sich der Behälterdeckel also in einem offenen Zustand befindet, zieht dies weitere positive Aspekte nach sich. Denn jener Deckelsensor sendet die von ihm erfasste Information überdies an die zentrale Steuereinheit. Diese sorgt dafür, dass der Desinfektionsbehälter nur dann mit Desinfektionsmittel versorgt wird, wenn sich der Deckel im geschlossenen, fluiddichten Zustand befindet. Der Deckelsensor trägt somit zur Betriebssicherheit bei und sorgt dafür, dass beispielsweise menschlich verursachte Fehler, wie das unvollständige Schließen des Desinfektionsbehälters, nachgebessert werden, bevor ihr negativer Effekt zum Tragen kommt. Somit ist die erfindungsgemäße Vorrichtung gegen unerwünschte Effekte, die beim Desinfektionsprozess auftreten können, gewappnet, wodurch neben der Betriebssicherheit auch der Komfort im Umgang mit der Anlage erhöht wird.

Gemäß einem anderen, ggf. unabhängig zu beanspruchenden Aspekt der vorliegenden Erfindung, hat der Desinfektionsbehälter einen Füllstandsensor, der dazu vorbereitet ist, das Volumen, das das Desinfektionsmittel im Behälterkörper einnimmt, zu erfassen. Auch dieser Füllstandsensor steht in Kommunikation mit der zentralen Steuereinheit, womit diese auch über die Information über den Füllstand des Desinfektionsmittels im Desinfektionsbehälter verfügt. So ist mittels des Füllstandsensors ein weiteres Merkmal realisiert, was die Betriebssicherheit und die Transparenz der einzelnen Prozesse erhöht. Denn mithilfe der Information über den Füllstand des Desinfektionsbehälters wird erreicht, dass unter anderen Faktoren abhängig von der Anzahl, dem Gewicht und der Größe der einzelnen Hilfsgegenstände, die sich im Inneren des Desinfektionsbehälters befinden, stets die optimale Desinfektionsmittelmenge vorhanden ist, die nötig ist, um einerseits die Hygieneanforderungen zu erfüllen und andererseits kein Desinfektionsmittel zu verschwenden. Der bereits erwähnte Vorteil der erhöhten betrieblichen Effizienz der erfindungsgemäßen Vorrichtung wird durch den Füllstandsensor also noch vergrößert.

Von Vorteil ist es zudem, wenn ein von dem Behälterkörper und dem Behälterdeckel definiertes Behältervolumen hinsichtlich seiner geometrischen Anordnung derart beschaffen ist, dass es keine Hinterschneidungen und/oder Toträume aufweist. Somit wird nach jedem Desinfektionsprozess eine gründliche und komplette Ausspülung des gesamten Desinfektionsbehälters ermöglicht, da das Behältervolumen keine Stellen aufweist, an denen sich Fluidreste anlagern können. Diese konstruktive Ausgestaltung trägt demnach dazu bei, dass die hohen Hygieneanforderungen eingehalten und/oder übertroffen werden. Dass bei einer solch klaren Geometrie des Innenvolumens die Hilfsgegenstände dennoch platzsparend angeordnet werden können, kann von einem Raumseparierer garantiert werden. Dieser ist beispielsweise formschlüssig, alternativ auch reibkraftschlüssig, in den Behälterkörper einsetzbar und kann so ausgestaltet werden, dass sämtliche Hilfsgegenstände in einer sinnvollen Anordnung im Inneren des Behälters angeordnet werden können. Der Raumseperierer wird von mehreren Trennwänden gebildet und ist hinsichtlich seiner Form variabel. Abhängig von den zu desinfizierenden Hilfsgegenständen, weist er ein symmetrisches oder asymmetrisches Profil auf.

Zudem zeichnet sich eine vorteilhafte Ausführungsform dadurch aus, dass die zentrale Steuereinheit eine derartige Einstellung aufweist, dass bei einem Befüllvorgang und bei einem Entleervorgang des Behältervolumens mit dem Desinfektionsmittel variable Retentionszeiten einstellbar sind. Daraus, dass diese Zeiten von der zentralen Steuereinheit gesteuert werden und somit variabel sind, folgt, dass unabhängig von einem Kontaminierungsgrad eines Hilfsgegenstandes stets eine vollständige Desinfektion erreicht wird. Die erfindungsgemäße Vorrichtung garantiert somit, dass unabhängig vom Betriebsfall höchste Qualitätsstandards eingehalten werden.

Der Desinfektionsprozess einer Dialysemaschine kann beispielhaft in folgende Betriebsphasen unterteilt werden:
- Ausspülen der Dialyseflüssigkeit
- Vorheizen
- Desinfektionsmittel ansaugen
- Desinfektionsmittel aufheizen
- heiße Desinfektionslösung zirkulieren
- Desinfektionslösung ausspülen und Maschine abkühlen.
Sämtliche Phasen werden von der zentralen Steuereinheit vorgegeben und sind mittels der Anordnung von Pumpen und Ventilen realisierbar. Es sei an dieser Stelle noch einmal angedeutet, dass jede der Phasen hinsichtlich ihrer Parameter, wie bspw. Zeit, Temperatur und Durchflussrate variabel ist. Weiterhin dienen die vorstehend genannten Betriebsphasen lediglich als Orientierung und sind nicht fest vorgeschrieben. So können weitere Betriebsphasen in den Desinfektionsprozess aufgenommen werden und/oder einzelne Phasen gestrichen werden.

Eine weitere vorteilhafte Ausführungsform besteht darin, dass der Desinfektionsbehälter eine Drucksteuerung aufweist, die derart ausgestaltet ist, dass sie den Füllstand des Desinfektionsbehälters erfasst. Somit stellt diese Drucksteuerung, die ebenfalls unter dem Einfluss der zentralen Steuereinheit steht, ein alternatives Konzept zu dem bereits offenbarten Füllstandsensor dar. So kann von Individualfall zu Individualfall entschieden werden, ob und welcher Sensor eingesetzt wird. So zeichnet sich die erfindungsgemäße Vorrichtung nicht nur im Betrieb durch die erhöhte Flexibilität aus, sondern auch in der Auswahl der einzelnen Komponenten.

### Figurenbeschreibung

Die Erfindung wird nachstehend anhand eines bevorzugten Ausführungsbeispiels unter Bezugnahme auf die begleitenden Figuren näher erläutert. Diese zeigen:
- Fig. 1: eine schematische Darstellung einer erfindungsgemäßen extrakorporalen Blutreinigungsanlage;
- Fig. 2: eine Schnittzeichnung durch einen erfindungsgemäßen Desinfektionsbehälter in einem geschlossenen Zustand;
- Fig. 3: eine Schnittzeichnung entlang einer weiteren Schnittebene durch einen erfindungsgemäßen Desinfektionsbehälter aus Fig. 2 in einem offenen Zustand;
- Fig. 4: eine Schnittzeichnung entlang einer weiteren Schnittebene durch einen erfindungsgemäßen Desinfektionsbehälter aus Fig. 2, 3.

Eine erfindungsgemäße extrakorporale Blutreinigungsanlage 1 weist mehrere Bestandteile auf. Hierzu zählt mindestens eine extrakorporale Blutreinigungsmaschine 2, die beispielsweise eine Dialyselösung bereitstellt. Der Aufbau einer extrakorporalen Blutreinigungsmaschine ist dem Fachmann beispielsweise aus der deutschen Offenlegungsschrift DE 103 19 220 A1 bekannt. Weiterhin offenbart die EP 1 491 222 A1 eine solche Maschine, sodass an dieser Stelle kein Bedarf besteht, auf den Aufbau einer extrakorporalen Blutreinigungsmaschine im Detail einzugehen.

Die erfindungsgemäße extrakorporale Blutreinigungsmaschine 2 kommuniziert mittels einer elektrischen Verbindung 20 mit einer zentralen Steuereinheit 19. Die zentrale Steuereinheit 19 hat Zugriff auf sämtliche Fluideinstellkomponenten der Anlage. Jene Fluideinstellkomponenten bezeichnen Ventile, wie Absperr- / Überdruck- / Rückschlag- / Druck- und Wegeventile sowie Pumpen jeglicher Art, die ein Fluid in einem Fluidsystem transportieren. Die Stellungen / Durchflussraten / Betriebsphasen der einzelnen Fluideinstellkomponenten werden von der zentralen Steuerneinheit 19 vorgeschrieben. Die zentrale Steuereinheit 19 steuert darüber hinaus die einzelnen Phasen der Blutbehandlungs- / Blutreinigungs- / Dialyseprozesse. Nach jenem Blutbehandlungsprozess durchläuft die Anlage einen Desinfektionsprozess. Dieser ist erfindungsgemäß ebenfalls von der zentralen Steuereinheit 19 steuerbar und schließt neben der Blutbehandlungsmaschine 2 auch einen Desinfektionsbehälter 12 und darin enthaltene Hilfsgegenstände mit ein.

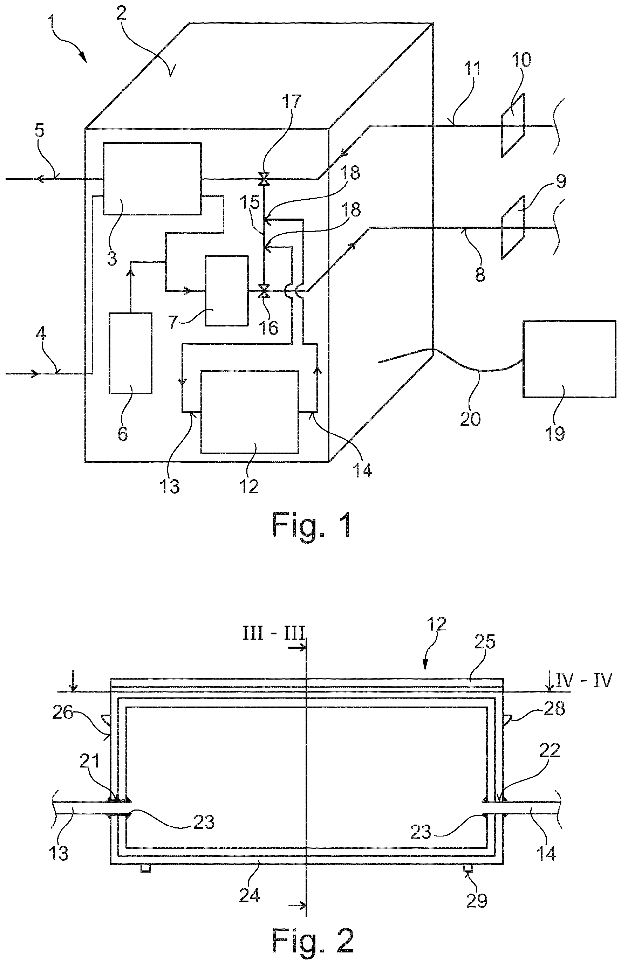

Figur 1 stellt einen schematischen Aufbau der extrakorporalen Blutreinigungsanlage 1 gemäß der vorliegenden Erfindung dar. Innerhalb der Blutreinigungsmaschine 2 weist die Anlage einen Wärmeübertrager 3 auf. Dieser eignet sich dazu, einem zufließenden Fluid Rekuperationswärme von einem abfließenden Fluid zu übertragen. Das zufließende Fluid tritt in die extrakorporale Blutreinigungsmaschine 2 über eine Zufuhrleitung 4 ein. Entsprechend verlässt das abfließende Fluid die Maschine über eine Abfuhrleitung 5.

Eine weitere zentrale Komponente der Blutreinigungsmaschine 2 ist eine Fluid-Aufbereitungskomponente 6. Diese steht mit der Zufuhrleitung 4 derart in Verbindung, dass sie das zufließende Fluid, beispielsweise (Osmose-)Wasser, zu einer Dialysierflüssigkeit aufbereitet. Die Dialysierflüssigkeit eignet sich, um eine Blutreinigung an einem Patienten vorzunehmen. Auch eine Bilanzierungseinrichtung 7, wie sie aus der vorstehend zitierten europäischen Offenlegungsschrift EP 1 491 222 A1 bekannt ist, ist für ein zuverlässiges Arbeiten der Blutreinigungsmaschine 2 von entscheidender Bedeutung. Die Dialysierflüssigkeit verlässt die Bilanzierungseinrichtung 7 über eine Frischdialysierflüssigkeitsleitung 8. Diese führt die Dialysierflüssigkeit bis zu einer ersten Anschlussventileinrichtung, vorzugsweise eine Dialysemembrankupplung 9. Mittels dieser ersten Dialysemembrankupplung 9 ist die Frischdialysierflüssigkeitsleitung 8 mit einem Eingang einer Membraneinheit (Dialysator) koppelbar, an welcher ein Blutbehandlungs- / Blutreinigungs- / Dialyseprozess stattfindet. Bei jener Membraneinheit (Dialysator) handelt es sich in der Regel um ein Einwegprodukt, weshalb in Figur 1 auf dessen detaillierte Darstellung verzichtet ist.

Eine zweite Anschlussventileinrichtung vorzugsweise eine Dialysemembrankupplung 10 verbindet eine verbrauchte Dialysierflüsskeitsleitung 11 mit einem Ausgang der Membraneinheit (Dialysator). So ermöglicht die verbrauchte Dialysierflüssigkeitsleitung 11 einen Rücktransport der verbrauchten Dialysierflüssigkeit. Nach einem Durchlaufen des Wärmetauschers 3 verlässt die verbrauchte Dialysierflüssigkeit die Blutreinigungsmaschine 2 über die Abfuhrleitung 5.

Um die Komplexität der Anlage überschaubar zu halten, sind in Figur 1 nur einige wenige Komponenten der extrakorporalen Blutbehandlungsmaschine 2 dargestellt. Weder bzgl. der Anzahl, noch bzgl. der Anordnung und Reihenfolge der Komponenten stellt Figur 1 eine einschränkende Ausführungsform dar. Hinsichtlich weiterer möglicher Bestandteile der extrakorporalen Blutbehandlungsmaschine 2 sei auf der vorstehend zitierten Stand der Technik verwiesen.

Erfindungsgemäß weist die extrakorporale Blutreinigungsanlage 1 zusätzlich zu der allgemein bekannten Blutbehandlungsmaschine 2 einen vorzugsweise externen Desinfektionsbehälter 12 zur Aufnahme von medizinischen Arbeitsgerätschaften auf, wie sie insbesondere bei der Blutbehandlung mittel der erfindungsgemäßen Blutbehandlungsanlage ggf. notwendig werden. Diesem ist mittels einer Behälterzufuhrleitung 13 Fluid zuführbar und mittels einer Behälterabfuhrleitung 14 Fluid abführbar. Erfindungsgemäß kann der Desinfektionsbehälter 12 entweder als externes Bauteil ausgestaltet sein, das mit der Blutbehandlungs-/reinigungsmaschine 2 vorzugsweise starr koppelbar ist oder er kann integral mit der Blutreinigungsmaschine 2 ausgestaltet sein. In Fig. 1 ist eine Blutbehandlungsanlage mit einem integrierten Desinfektionsbehälter 12 dargestellt, wobei in diesem Fall eine Beladungstür oder Beladungsdeckel am Gehäuse der Blutreinigungsmaschine 2 vorgesehen ist, über welche der Desinfektionsbehälter 12 unmittelbar beladen/entladen oder dieser aus der Blutreinigungsmaschine 12 für ein Beladen/Entladen entnehmbar ist.

Weiterhin ist es erfindungsgemäß vorgesehen, den Desinfektionsbehälter 12 mit einer allgemein bekannten Spülbrücke 15 der Blutreinigungsmaschine 2 und/oder mit einem eigenen, autarken Desinfektionskreislauf zu koppeln bzw. zu versehen. Figur 1 zeigt die Ausführungsform mittels der Spülbrücke 15. Ein detaillierter Aufbau einer Spülbrücke ist dem Fachmann aus der DE 10 2013 107 323 A1 bekannt, weshalb dieser im Rahmen dieser Anmeldeschrift nicht weiter vertieft wird.

Die Spülbrücke 15 ist eine Art Kurzschluss zwischen der Frischdialysierflüssigkeitsleitung 8 und der verbrauchten Dialysierflüssigkeitsleitung 11. Während des Dialyseprozesses ist die Spülbrücke 15 mittels Sperrventilen 16, 17 vom Dialysierflüssigkeitskreislauf getrennt. Erst im Falle eines Desinfektionsprozesses werden die Sperrventile 16, 17 geöffnet, damit die Spülbrücke 15 von einem Desinfektionsmittel durchfließbar ist / durchflossen wird. Zusätzlich kann auch an der Frischdialysierflüssigkeitsleitung 8 und der verbrauchten Dialysierflüssigkeitsleitung 11 jeweils ein Ventil ausgebildet sein, um die Membraneinheit (Dialysator) während des Desinfektionsvorgangs besser von dem Fluidkreislauf abzukoppeln. Anhand von Spülbrückenanschlüssen 18 sind die Behälterzufuhrleitung 13 und die Behälterabfuhrleitung 14 mit der Spülbrücke 15 koppelbar bzw. gekoppelt. Ist ein Desinfektionsbehälter für einen Desinfektionsvorgang an der Spülbrücke 15 angeschlossen, so wird zusätzlich der Fluidfluss durch die Spülbrücke 15 mit einem ein Ventil 15a blockiert, sodass das Fluid durch den Desinfektionsbehälter (stellt nunmehr die ggf. externe Spülbrücke dar) geleitet wird.

Alternativ zu den Sperrventilen 17 und 16 kann auch jeweils an der Abzweigung zu der Spülbrücke 15 von der Fluidleitung eine Weiche vorgesehen sein, mit der sich der Fluidfluss umschalten lässt. Zu diesem Zweck könnte alternativ auch ein Mehrwegehahn an der Abzweigung der Spülbrücke 15 von der Fluidleitung vorgesehen sein.

Das Umschalten des Fluidflusses über beispielsweise ein Ventil, einen Mehrwegehahn oder eine sonstige Weiche kann hierbei manuell oder automatisch erfolgen. Beispielsweise könnte ein Benutzer händisch den Mehrwegehahn umstellen. Alternativ könnte die Umstellung allerdings auch automatisch erfolgen, beispielsweise nach der Eingabe eines entsprechenden Befehls von dem Benutzer in die Steuereinheit. Auch könnte die zentrale Steuereinheit aus den Betriebsdaten der Blutbehandlungsanlage 1 erfassen, dass ein Desinfektionsprozess nötig wäre und vollautomatisch bzw. ohne eine weitere Benutzereingabe die Weichen bzw. Ventile bzw. Mehrwegehähne umstellen und den Desinfektionsprozess einleiten.

Wird ein Desinfektionsbehälter verwendet, welcher nicht permanent in der Blutbehandlungsanlage integriert ist, besteht zudem die Möglichkeit, dass das physische Ankoppeln des Desinfektionsbehälters an die Anschlüsse der Blutbehandlungs-/reinigungsmaschine 2 per se nach dem Prinzip eines Plug- and-Play-Anschlusses eine Umleitung des Reinigungsfluids in den externen Desinfektionsbehälter 12 bewirkt.

Sobald der Desinfektionsbehälter 12 über die interne Spülbrücke 15 mit einem Desinfektionszyklus verbunden ist, fließt das Desinfektionsmittel, von nicht dargestellten Pumpen angetrieben, durch den Desinfektionsbehälter 12. Hierbei findet eine Desinfektion/Reinigung der innerhalb des Desinfektionsbehälters 12 angeordneten Hilfsgegenstände statt. Die einstellbaren Parameter, wie die Durchflussrate und -zeit, sowie die Temperatur des Desinfektionsmittels, werden von Seiten der zentralen Steuereinheit 19 derart angepasst, dass die Hilfsmittel vollständig desinfizierbar sind / desinfiziert werden. Mittels einer im Rahmen dieser Offenlegungsschrift nicht weiter vertieften Sensorik kann während des Desinfektionsprozesses überprüft werden, inwieweit der Kontaminierungsgrad der Hilfsgegenstände über die Zeit abnimmt.

Die zentrale Steuereinheit 19 ist ebenfalls Teil der extrakorporalen Blutreinigungsanlage 1 und kommuniziert mit der Blutreinigungsmaschine 2 vorzugsweise über die elektrische Verbindung 20. So ist die zentrale Steuereinheit 19 derart einstellbar / programmierbar / einrichtbar, dass die extrakorporale Blutreinigungsanlage hinsichtlich des Fluidtransports variabel ist und flexibel reagieren kann. Denn neben den in Figur 1 dargestellten Sperrventilen 16, 17 verfügt die Anlage über weitere Ventileinheiten, wie vorstehend angedeutet, die von der zentralen Steuereinheit steuerbar sind / gesteuert werden. Aus Gründen der Übersichtlichkeit sind jene weiteren Ventileinheiten, die dem Fachmann aus dem vorstehend zitierten Stand der Technik bekannt sind, nicht dargestellt.

Die begleitenden Figuren 2, 3 und 4 stellen allesamt einen Desinfektionsbehälter 12 dar, der als externes Bauteil mit der Blutreinigungsmaschine 2 koppelbar ist.

Zu einem Ausführungsbeispiel, in dem der Desinfektionsbehälter 12 eine näherungsweise quaderförmige Grundfläche hat und an einer der beiden längeren Seiten mit der Blutreinigungsmaschine 2 in Kontakt steht, stellt Figur 2 einen Schnitt entlang der Frontalebene dar. Dieser Schnitt ist so angesetzt, dass sowohl die Behälterzufuhrleitung 13, als auch die Behälterabfuhrleitung 14 in der Schnittebene liegen. Die Behälterzufuhrleitung 13 ist mittels eines Zufuhranschlusses 21 mit dem Desinfektionsbehälter 12 verbunden. Analog gilt dies für die Behälterabfuhrleitung 14, die mittels eines Abfuhranschlusses 22 mit dem Desinfektionsbehälter 12 verbunden ist. Jene Verbindungen zwischen den Leitungen und den Anschlüssen können von verschiedenen Kupplungselementen, wie integrierten Kupplungen oder Hansen-Kupplungen, vorgenommen werden. Dichtelemente 23 bilden einen Teil des Zufuhr- 21 bzw. Abfuhranschlusses 22. Die Dichtelemente 23 sorgen für eine fluiddichte Verbindung zwischen der Behälterzufuhr- 13 bzw. der Behälterabfuhrleitung 14 und dem Desinfektionsbehälter 12.

Das in Figur 2 dargestellte Ausführungsbeispiel weist eine Anordnung auf, bei der der Zufuhranschluss 21 und der Abfuhranschluss 22 auf der gleichen Höhe an gegenüberliegenden Seiten angeordnet sind. Hierzu sei erwähnt, dass die erfindungsgemäße Vorrichtung keineswegs auf jene Ausführungsform beschränkt ist. So ist es durchaus denkbar, dass der Zufuhranschluss 21 höher angeordnet ist als der Abfuhranschluss 22 oder andersherum. Weiterhin ist die Vorrichtung hinsichtlich der Anordnung der Anschlüsse flexibel. So sind der Zufuhranschluss 21 und der Abfuhranschluss 22 in einer anderen Ausführungsform an der gleichen oder an benachbarten Seitenwänden angeordnet. Dies kann hinsichtlich der Strömungseigenschaften des Desinfektionsmittels und der daraus resultierenden Wirbelströmungen innerhalb des Desinfektionsbehälters 12 wünschenswert sein, um einen effizienteren Desinfektionsprozess zu forcieren. Auch eine Anordnung der Anschlüsse in einem Behälterboden 24 oder einem Behälterdeckel 25 kann angestrebt werden. Dies kann sich, je nach der Anordnung der Hilfsgegenstände innerhalb des Desinfektionsbehälters 12 positiv auf die Retentionszeiten auswirken.

Behälterwände 26 bilden zusammen mit dem Behälterboden 24 einen Behälterkörper 27. Dieser kann vom Behälterdeckel 25 fluiddicht verschlossen werden. In dem von den Figuren 2 bis 4 dargestellten Ausführungsbeispiel handelt es sich bei dem Behälterdeckel 25 um einen Klappdeckel, der einseitig gelagert ist. Darüber hinaus sind auch zweiteilige, beidseitig gelagerte Klappdeckel und/oder Schiebevorrichtungen und/oder separate Deckelvorrichtungen als Verschlussmechanismus möglich. Hinsichtlich seiner geometrischen Form ist der Behälterdeckel 25 variabel gestaltbar und nicht auf die überwiegend plane Form aus den Figuren beschränkt. So kann eine glocken-/beulenförmige und/oder konvexe Gestaltung wünschenswert sein, um das Volumen des Behälterkörpers 27 zu vergrößern und somit mehr Hilfsgegenstände aufnehmen zu können.

Um die Handhabung mit dem Desinfektionsbehälter 12 zu vereinfachen, ist es möglich, an den Behälterwänden 26 Griffe 28 anzuordnen. Diese haben ebenso wie Standfüße 29 im Betrieb keine unmittelbare Funktion. Vielmehr eignen sie sich dazu, beispielsweise den Prozess des Behälteraustauschens zu erleichtern, da sie einem Monteur die Möglichkeit geben, den Desinfektionsbehälter 12 leicht abzustellen und zu transportieren/hochzuheben. Bezüglich der Griffe 28 bietet es sich an, sie aus einem anderen Material / anderen Materialien zu fertigen, als den restlichen Behälter. So wird vermieden, dass die Griffe 28 von einem Desinfektionsmittel, das im Desinfektionsprozess Temperaturen von 70°C bis 90°C erreichen kann, erhitzt werden und somit stets anfassbar bleiben. Für die Materialien / das Material des Desinfektionsbehälters 12 bieten sich vor allem Kunststoffe an.

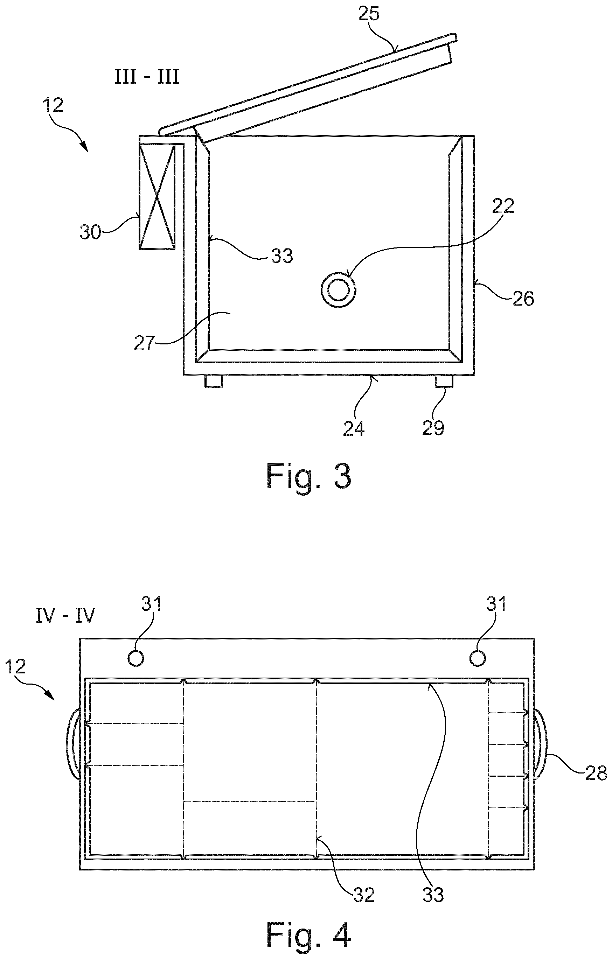

Der Schnitt durch die Frontalebene in Figur 2 deutet ebenfalls die beiden Schnittebenen aus Figur 3 und Figur 4 an. Für deren Verdeutlichung werden die römischen Zahlen III respektive IV verwendet.

Figur 3 stellt demnach einen Schnitt in der Sagittalebene des Desinfektionsbehälters 12 aus Figur 2 dar. An der der Dialysemaschine 2 zugewandten Seite, weist der Behälterkörper 27 Verbindungselemente 30 auf. Diese sind ein zentraler Bestandteil des Desinfektionsbehälters 12, da sie es in der vorstehend erwähnten externen Ausführung ermöglichen, dass der Desinfektionsbehälter 12 starr mit der Dialysemaschine 2 verbindbar ist. Die Verbindungselemente 30 rufen eine form- und/oder reibkraft- und/oder stoffschlüssige Verbindung der beiden Komponenten "Desinfektionsbehälter 12" und "Dialysemaschine 2" hervor. Hinsichtlich ihrer Anordnung sind sie keinesfalls auf die in Figur 3 angedeutete Form beschränkt. Sie können an jeder der Behälterwände 26, wahlweise auch an mehreren Behälterwänden 26 und/oder auch dem Behälterdeckel 25 und/oder dem Behälterboden 24 ausgebildet sein. Vorzugsweise sind die Verbindungselemente 30 derart ausgestaltet, dass sie eine seitliche Montage an der Dialysemaschine 2 ermöglichen.

In der in Figur 2 dargestellten Ausführungsform ist klar ersichtlich, dass der Behälterkörper 27 keinerlei Toträume und/oder Hinterschneidungen aufweist, wodurch eine gründliche, bakterienfreie Ausspülung des Desinfektionsbehälters 12 ermöglicht wird.

Figur 4 zeigt einen Schnitt durch die Transversalebene des Behälterkörpers 27. Der Behälterdeckel 25 ist in Figur 4 nicht dargestellt. Eine Deckelbefestigung 31 ist in Figur 4 beispielhaft mittels zweier Öffnungen realisiert. Diese können den Behälterdeckel 25 fixieren, während eine nicht dargestellte Lagerung für die Rotationsbeweglichkeit des Behälterdeckels 25 zuständig ist. Die Deckelbefestigung 31 ist lediglich schematischer Natur und schreibt keine einschränkende Ausführungsform vor. Schematisch angedeutete Trennwände 32 haben die Funktion, den Hilfsgegenständen eine Fixierung zu bieten. Sie können frei angeordnet sein und sind mit einer Behälterinnenwand 33 beispielsweise formschlüssig, alternativ auch reibkraft- und/oder stoffschlüssig in Verbindung.

### Bezugszeichenliste

- 1.: extrakorporale Blutreinigungsanlage
- 2.: extrakorporale Blutreinigungsmaschine
- 3.: Wärmetauscher
- 4.: Zufuhrleitung
- 5.: Abfuhrleitung
- 6.: Aufbereitungskomponente
- 7.: Bilanzierungseinrichtung
- 8.: Frischdialysierflüssigkeitsleitung
- 9.: erste Dialysemembrankupplung
- 10.: zweite Dialysemembrankupplung
- 11.: verbrauchte Dialysierflüssigkeitsleitung
- 12.: Desinfektionsbehälter
- 13.: Behälterzufuhrleitung
- 14.: Behälterabfuhrleitung
- 15.: Spülbrücke
- 16.: Sperrventil
- 17.: Sperrventil
- 18.: Spülbrückenanschluss
- 19.: zentrale Steuereinheit
- 20.: elektrische Verbindung
- 21.: Zufuhranschluss
- 22.: Abfuhranschluss
- 23.: Dichtelement
- 24.: Behälterboden
- 25.: Behälterdeckel
- 26.: Behälterwand
- 27.: Behälterkörper
- 28.: Griffe
- 29.: Standfüße
- 30.: Verbindungselemente
- 31.: Deckelbefestigung
- 32.: Trennwand
- 33.: Behälterinnenwand

## Patentansprüche

1. Extrakorporale Blutreinigungsanlage (1) mit
- mindestens einer extrakorporalen Blutreinigungsmaschine (2) vorzugsweise Dialysemaschine, welche dazu vorbereitet ist, nach einem Blutreinigungsprozess einen zentral gesteuerten Desinfektionsprozess zu durchlaufen, sowie mit
- einem internen Fluidleitungssystem, welches zumindest während des Desinfektionsprozesses von einem Desinfektionsmittel durchfließbar ist,
einem vorzugsweise externen Desinfektions- oder Reinigungsbehälter (12), der dafür vorgesehen ist, zusätzliche Hilfsgegenstände aufzunehmen und der zumindest während des Desinfektionsprozesses über mindestens einen Behälteranschluss derart an das interne Fluidleitungssystem anschließbar oder angeschlossen ist, dass er während des Desinfektionsprozesses mitdesinfizier-/reinigbar ist oder mitdesinfiziert/-gereinigt wird,
**dadurch gekennzeichnet, dass** der Desinfektionsbehälter (12) über eine Behälterzufuhrleitung (13) und eine Behälterabfuhrleitung (14) mit einer einen Dialysator der Blutreinigungsmaschine (2) kurzschließenden Spülbrücke (15) verbindbar ist, welche ein integraler Bestandteil des Fluidleitungssystems der Blutreinigungsmaschine (2) ist.

2. Extrakorporale Blutreinigungsanlage (1) nach Anspruch 1,
**dadurch gekennzeichnet, dass**
an der Blutbehandlungsmaschine (2) und /oder dem Desinfektionsbehälter (12) Fluidleitungsmittel (16,17) vorgesehen sind, mittels derer der Fluidfluss in dem Fluidleitungssystem entweder geschlossen intern in der Blutreinigungsmaschine (2) zirkuliert werden kann oder vorzugsweise während des Desinfektionsprozesses zusätzlich auch durch den Desinfektionsbehälter (12) geleitet werden kann.

3. Extrakorporale Blutbehandlungsanlage (1) nach Anspruch 2,
**dadurch gekennzeichnet, dass**
die Fluidleitungsmittel (16,17) jeweils eine Weiche, ein Ventil oder ein Mehrwegehahn sind.

4. Extrakorporale Blutbehandlungsanlage (1) nach Anspruch 2 oder 3,
**dadurch gekennzeichnet, dass**
die Fluidleitungsmittel (16,17) manuell oder automatisch zum Umschalten des Fluidflusses zwischen der geschlossenen internen Fluidzirkulation in der Blutreinigungsmaschine (2) und der zumindest teilweisen Umleitung des Fluidflusses durch den Desinfektionsbehälter (12) vorzugsweise während des Desinfektionsprozesses ansteuerbar sind.

5. Extrakorporale Blutreinigungsanlage (1) nach einem der vorhergehenden Ansprüche,
**dadurch gekennzeichnet, dass**
die Behälterzufuhrleitung (13) und die Behälterabfuhrleitung (14) des Desinfektionsbehälters (12) einen Kreislauf mit einer eigenen, mit dem internen Fluidleitungssystem in Verbindung stehenden Desinfektionsmittelversorgung bilden.

6. Extrakorporale Blutreinigungsanlage (1) nach einem der vorstehenden Ansprüche,
**dadurch gekennzeichnet, dass**
der Desinfektionsbehälter (12) solche mechanischen Verbindungselemente (30) aufweist, dass er starr mit der Blutreinigungsmaschine (2) koppelbar ist.

7. Extrakorporale Blutreinigungsanlage (1) nach einem der vorstehenden Ansprüche,
**dadurch gekennzeichnet, dass**
der Desinfektionsbehälter (12) integral mit der Blutreinigungsmaschine (2) ausgebildet ist.

8. Extrakorporale Blutreinigungsanlage (1) nach einem der vorstehenden Ansprüche,
**dadurch gekennzeichnet, dass**
der Desinfektionsbehälter (12) einen Deckelsensor hat, der dazu vorbereitet ist, zu erfassen, ob ein Behälterdeckel (25) fluiddicht auf einem Behälterkörper (27) anliegt, sich der Behälterdeckel (25) also in einem geschlossenen Zustand befindet oder ob der Behälterdeckel (25) nicht fluiddicht auf dem Behälterkörper (27) anliegt, sich der Behälterdeckel (25) also in einem offenen Zustand befindet.

9. Extrakorporale Blutreinigungsanlage (1) nach einem der vorstehenden Ansprüche,
**dadurch gekennzeichnet, dass**
der Desinfektionsbehälter (12) einen Füllstandsensor hat, der dazu vorbereitet ist, das Volumen, das das Desinfektionsmittel im Behälterkörper (27) einnimmt, zu erfassen.

## Claims

1. An extracorporeal blood purification system (1) comprising
- at least one extracorporeal blood purification machine (2), preferably dialysis machine, which is prepared for undergoing a centrally-controlled disinfection process following a blood purification process, and comprising
- an internal fluid line system through which a disinfectant may flow at least during the disinfection process,
- a preferably external disinfection or purification case (12) being adapted for receiving additional accessories and being connectable or connected, at least during the disinfection process, via at least one case port to the internal fluid line system so that it is adapted to be also disinfected/purified or is also disinfected/purified during the disinfection process, **characterized in that** the disinfection case (12) is connectable via a case supply line (13) and a case drain line (14) to a flushing bridge (15) which short-circuits a dialyser of the blood purification machine (2) and which is an integral part of the fluid line system of the blood purification machine (2).

2. The extracorporeal blood purification system (1) according to claim 1,
**characterized in that**
fluid guiding means (16, 17) are provided on the blood treatment machine (2) and/or the disinfection case (12) for circulating the fluid flow in the fluid guiding system either in a closed internal manner within the blood purification machine (2) or for guiding the fluid flow additionally also through the disinfection case (12) preferably during the disinfection process.

3. The extracorporeal blood treatment system (1) according to claim 2,
**characterized in that**
the fluid guiding means (16, 17) constitute a respective switch point, a valve or a multiple-way cock.

4. The extracorporeal blood treatment system (1) according to claim 2 or 3,
**characterized in that**
the fluid guiding means (16, 17) are adapted to be controlled manually or automatically for changing over the fluid flow between the closed internal fluid circulation within the blood purification machine (2) and the at least partial bypass of the fluid flow through the disinfection case (12) preferably during the disinfection process.

5. The extracorporeal blood purification system (1) according to one of the preceding claims,
**characterized in that**
the case supply line (13) and the case drain line (14) of the disinfection case (12) form a circuit having a separate disinfectant supply being communicated with the internal fluid line system.

6. The extracorporeal blood purification system (1) according to one of the preceding claims,
**characterized in that**
the disinfectant case (12) includes mechanical connecting elements (30) so that it can be rigidly coupled to the blood purification machine (2).

7. The extracorporeal blood purification system (1) according to one of the preceding claims,
**characterized in that**
the disinfection case (12) is formed integrally with the blood purification machine (2).

8. The extracorporeal blood purification system (1) according to one of the preceding claims,
**characterized in that**
the disinfection case (12) includes a lid sensor provided for detecting whether a case lid (25) is in fluid-tight contact with a case body (27) and hence the case lid (25) is in a closed state, or whether the case lid (25) is not in fluid-tight contact with the case body (27) and hence the case lid (25) is in an open state.

9. The extracorporeal blood purification system (1) according to one of the preceding claims,
**characterized in that**
the disinfection case (12) includes a filling level sensor provided for detecting the volume occupied by the disinfectant inside the case body (27).

## Revendications

1. Installation de purification du sang extracorporelle (1) comprenant
- au moins une machine de purification du sang extracorporelle (2) de préférence une machine de dialyse, laquelle est préparée pour suivre après une procédure de purification du sang une procédure de désinfection commandée de manière centralisée, ainsi que
- un système d'acheminement de fluide intérieur, lequel peut être traversé par un désinfectant au moins pendant la procédure de désinfection,
un récipient de désinfection ou de purification (12) de préférence extérieur, qui est prévu pour recevoir des objets auxiliaires supplémentaires et qui peut être raccordé ou branché au moins pendant la procédure de désinfection sur au moins un raccord de récipient au système d'acheminement de fluide intérieur, de sorte qu'il peut être également désinfecté/nettoyé ou est également désinfecté/nettoyé pendant la procédure de désinfection,
**caractérisée en ce que** le récipient de désinfection (12) peut être relié par l'intermédiaire d'une conduite d'alimentation de récipient (13) et d'une conduite d'évacuation de récipient (14) avec une passerelle de rinçage (15) de mise en court-circuit d'un dialyseur de la machine de purification du sang (2), laquelle fait partie intégrante du système d'acheminement de fluide de la machine de purification du sang (2).

2. Installation de purification du sang extracorporelle (1) selon la revendication 1,
**caractérisée en ce que**
des moyens d'acheminement de fluide (16,17) sont prévus au niveau de la machine de traitement du sang (2) et/ou du récipient de désinfection (12), au moyen desquels l'écoulement de fluide peut circuler dans le système d'acheminement de fluide soit de manière fermée à l'intérieur de la machine de purification du sang (2), soit peut être acheminé de préférence pendant la procédure de désinfection en outre également à travers le récipient de désinfection (12).

3. Installation de purification du sang extracorporelle (1) selon la revendication 2,
**caractérisée en ce que**
les moyens d'acheminement de fluide (16, 17) sont respectivement un aiguillage, une soupape ou un robinet de distribution multiple.

4. Installation de purification du sang extracorporelle (1) selon la revendication 2 ou 3,
**caractérisée en ce que**
les moyens d'acheminement de fluide (16, 17) peuvent être commandés manuellement ou automatiquement pour commuter l'écoulement de fluide entre la circulation de fluide interne fermée dans la machine de purification du sang (2) et la dérivation au moins partielle de l'écoulement de fluide à travers le récipient de désinfection (12) de préférence pendant la procédure de désinfection.

5. Installation de purification du sang extracorporelle (1) selon l'une quelconque des revendications précédentes,
**caractérisée en ce que**
la conduite d'alimentation de récipient (13) et la conduite d'évacuation de récipient (14) du récipient de désinfection (12) forment un circuit avec un approvisionnement en désinfectant propre étant en communication avec le système d'acheminement de fluide intérieur.

6. Installation de purification du sang extracorporelle (1) selon l'une quelconque des revendications précédentes,
**caractérisée en ce que**
le récipient de désinfection (12) présente de tels éléments de liaison mécaniques (30) qu'il peut être couplé de manière rigide à la machine de purification du sang (2).

7. Installation de purification du sang extracorporelle (1) selon l'une quelconque des revendications précédentes,
**caractérisée en ce que**
le récipient de désinfection (12) est formé d'une seule pièce avec la machine de purification du sang (2).

8. Installation de purification du sang extracorporelle (1) selon l'une quelconque des revendications précédentes,
**caractérisée en ce que**
le récipient de désinfection (12) possède un capteur de couvercle, qui est préparé à détecter si un couvercle de récipient (25) repose de manière étanche aux fluides sur un corps de récipient (27), le couvercle de récipient (25) se trouve donc dans un état fermé ou si le couvercle de récipient (25) ne repose pas de manière étanche aux fluides sur le corps de récipient (27), le couvercle de récipient (25) se trouve donc dans un état ouvert.

9. Installation de purification du sang extracorporelle (1) selon l'une quelconque des revendications précédentes,
**caractérisée en ce que**
le récipient de désinfection (12) possède un capteur de niveau de remplissage qui est préparé à détecter le volume qu'occupe le désinfectant dans le corps de récipient (27).
